# EUROPEAN PATENT SPECIFICATION

(11) **EP 0 429 906 B1**
(45) Date of publication and mention of the grant of the patent: **01.03.1995**
(21) Application number: 90121330.6
(22) Date of filing: 07.11.1990
(51) Int. Cl.: G02B 23/26, A61B 1/00, G02B 23/16

(54) **Direct vision / side vision exchangeable endoscope**
Endoskop mit austauschbarer Direktsicht-/Seitsichtbeobachtung
Endoscope avec dispositif de vision directe/latéral échangeable

(30) Priority: 09.11.1989 JP 130112/89 U
(43) Date of publication of application: 05.06.1991
(73) Proprietor: MACHIDA ENDOSCOPE CO., LTD, Bunkyo-ku, Tokyo (JP)
(72) Inventor: Oku, Toshio, Bunkyo-ku, Tokyo (JP)
(74) Representative: Koch, Günther, Dipl.-Ing.

(56) References cited:
- DE-A- 3 439 876
- FR-B- 2 356 037
- JP-A- 1 137 221
- US-A- 1 413 024
- US-A- 1 656 118
- US-A- 3 434 775
- US-A- 4 787 370

## Description

The present invention relates to an encoscope in which a direct-vision attachment and a side-vision attachment can be exchangeably attached as the attachment on the top end of the endoscope.

An endoscope in which a direct-vision attachment and a side-vision attachment is exchangeably attached to an object lens on the top end of the endoscope so that one endoscope can be used as both of direct-vision and side-vision endoscopes has been known, and the endoscope of this type is disclosed for example, in Japanese Laid-Open Utility Model Publication No. 62-184515.

In this conventional technique, the attachment is attached and fixed to the top end hard portion of the endoscope by means of a pin inserted in the top end hard portion or a thread formed on the top end hard portion.

However, the conventional fixing means is insufficient in that the fixing means is loosened by vibration or heat in an object to be examined, in which the endoscope is inserted, and there is a risk of falling of the object lens.

For example, in the case where the interior of a jet engine is inspected by the endoscope, falling of the attachment in the engine results in occurrence of a grave accident. Similarly, there is a risk of falling of the attachment when the body cavity is examined by the endoscope.

This invention relates more particularly to an endoscope comprising a direct-vision attachment and a side-vision attachment as specified in the preamble of claim 1 and claim 3, respectively. An endoscope like this is disclosed in JP-A-1-137 211, figure 4. In the fixed position the anchoring ring engages with its threaded nut an outer thread of the top end hard portion of the endoscope. The threaded nut of the attachment in the coupled position is unscrewed from the forward thread of the top end. This means that the attachment becomes loose as soon as the anchoring ring is unscrewed, e.g. by vibration. The attachment is not longer aligned with the endoscope and is no more operable. There is in fact an engagement between the outer thread of the top end and the threaded nut of the attachment which prevents falling down of the attachment. However, the thread engagement is relatively loose so that the attachment easily can be unscrewed from the top end, e.g. by vibration.

Therefore it is a primary object of the present invention to provide a direct vision / side vision exchangeable endoscope in which the attachment is kept screwed even if the anchoring ring comes off, and falling of the attachment is prevented wherein
the effect of preventing falling of the attachment is similarly attained, even if the anchoring ring is freely set on the top end hard portion and wherein
to an operator can be informed of coming-off of the anchoring ring and occurrence of an accident can be prevented, in case of the side-vision attachment.

The above technical problem is solved by the features of the characterizing portion of claims 1 and 3, respectively. Further developments are specified in claims 2 and 4, respectively.

In the endoscope having the above-mentioned structure, the attachment is screwed to the top end hard portion of the endoscope by means of the threaded nut, and the anchoring ring is further screwed, whereby a double-lock structure can be formed and falling of the attachment can be assuredly prevented.

### Brief Description of the Drawings

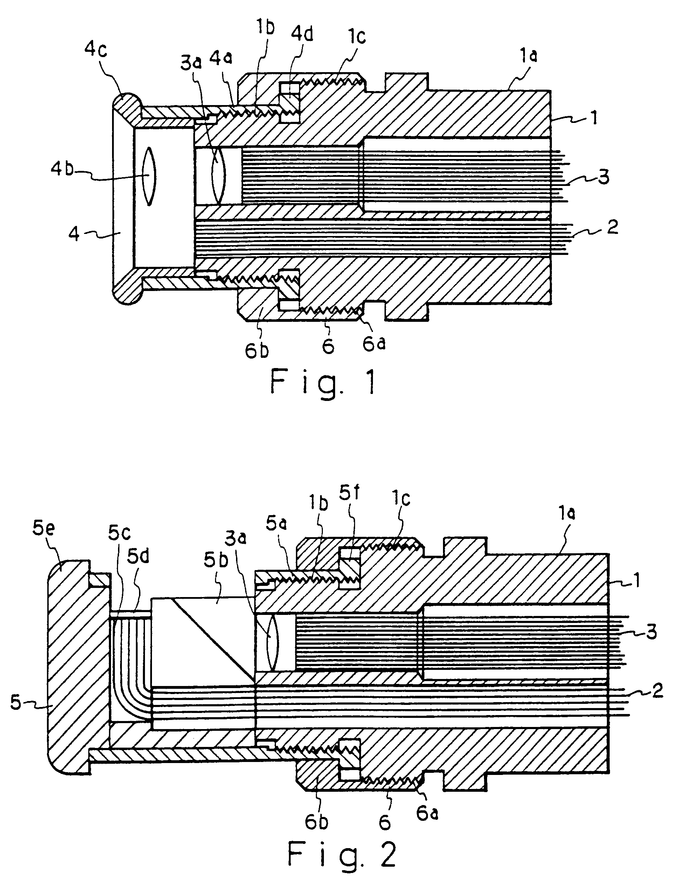

Fig. 1 is a sectional view illustrating the first embodiment in which the direct-vision attachment is fitted.

Fig. 2 is a sectional view illustrating the first embodiment in which the side-vision attachment is fitted.

Fig. 3 is a sectional view illustrating a main part of a second embodiment.

### Detailed Description of the Preferred Embodiments

Enbodiments of the present device will now be described with reference to the accompanying drawings.

In the drawings, reference numeral 1 represents a top end hard portion of the endoscope, and at least a light guide 2 and an image guide 3 are passed through the top end hard portion 1 and a lens system 3a is arranged on the top end of the image guide 3. If necessary, an air or water conduit and a forceps-introducing tube passed through the top end hard portion 1, and a step 1a is formed at the rear part of the outer circumference of the top end hard portion 1. This top end hard portion 1 is connected to a flexible tube, not shown in the drawings, of the endoscope, and an endo portion of an outer casing covering the flexible tube also covers this step 1a.

Reference numeral 1b represents a first thread and reference numeral 1c represents a second thread, and the threads 1b and 1c are inverse to each other and the diameter of the second thread 1c is larger than the diameter of the first thread 1b.

Reference numeral 4 represents a direct-vision attachment, and a threated nut 4a to be engaged with the second thread 1c is formed on the inner side of the rear end of the attachment 4. According to need, an object lens 4b is also arranged on the attachment 4. Reference numerals 4c and 4d represent flanges formed on the outer circumference at both the ends of the attachment 4.

Reference numeral 5 represents a side-vision attachment, and a threated nut 5a to be engaged with the first thread 1b is formed on the inner side of the rear end of the attachment 5 and a viewing window is formed on the side face of the attachment 5. A light-refracting means comprising a mirror, a prism and the like is arranged to confront this side face. A glass or lens window 5d is formed on the top end face of a light guide 5c. Reference numerals 5e and 5f represent flanges formed on the outer circumference at both the ends.

Reference numeral 6 represents an anchoring ring, and a threaded nut 6a to be engaged with the second thread 1c is formed on the inner side of the top end and the anchoring ring 6 is fitted between both the flanges of the attachment so that the ring 6 can move in the front-rear direction and can turn relatively to the axis. An anchoring portion 6b is formed on the inner side of the rear end of the ring 6.

According to the embodiment having the above-mentioned structure, the directe-vision or side-vision attachment is selected and attached to the top end hard portion 1. Since the function is almost the same irrespectively of the kind of the selected attachment, the function will now be described with reference to the side-vision attachment 5.

At first, the threaded nut 5a of the side-vision attachment 5 is screwed and attached to the first thread 1b of the top end hard portion 1 so that the refracting means 5b confronts the image guide 3 of the top end hard portion 1 and the light guide 5c confronts the light guide 2.

Then, the threaded nut 6a of the anchoring ring 6 fitted in the side-vision attachment 4 is engaged with the second thread 1c of the top end hard portion 1, whereby the anchoring portion 6b anchors the flange 5f and the attachment 5 is further fastened.

Thus, the threaded nut 5a of the attachment 5 is engaged with the first thread 1b of the top end hard portion 1 and the attachment 5 is integrated with the top end hard portion 1, and when the anchoring ring 6 is fitted and screwed, the integration degree is enhanced. At this point, the screwing directions of the threaded nut 5a of the attachment 5 and the threaded nut 6a of the anchoring ring 6 are inverse to each other.

When the attachment thus attached is dismounted, the anchoring ring 6 is first dismounted and the attachment 5 is turned.

Since the direct-vision attachment 4 is attached and dismounted substantially in the same manner as described above with respect to the attachment 5, the explanation is omitted.

In the above-mentioned first embodiment, the first thread 1b and the second thread 1c are inverse to each other. In a second embodiment not shown in the drawings, the screw direction is the same in both of the first and second thread, but the threads are different in the screw pitch, and corresponding threads are formed on the respective attachments and the anchoring ring. Also in this embodiment, there can be attained effects similar to those attained in the first embodiment.

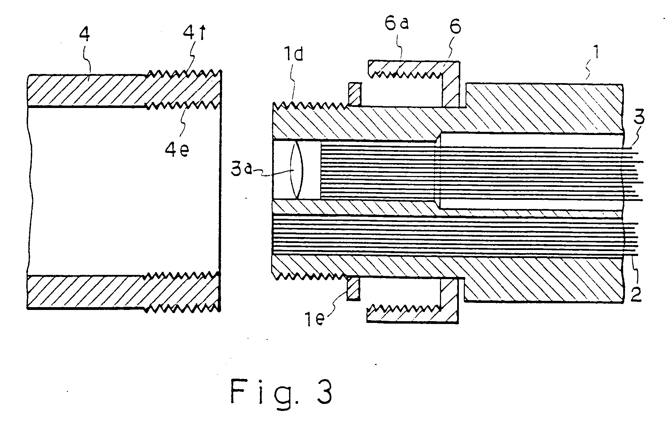

Fig. 3 is a sectional view showing a main part of a third embodiment. A thread 1d is formed on the top end of a top end hard portion 1, and then, through a flange le, an anchoring ring 6 is fitted, as in the first embodiment, so that the anchoring ring 6 can move in the front-rear direction and can turn in the axial direction. A screw 6a is formed on the inner side of the anchoring ring 6, and the screw direction of this threaded nut 6a is inverse to the screw direction of the thread 1d or both the threads are the same in the screw direction but different in the screw pitch.

Since direct-vision and side-vision attachments are similar to each other, only the direct-vision attachments will now be described.

A threaded nut 4e to be engaged with the thread 1d is formed on the inner circumference of the rear end of the direct-vision attachment 4, and a thread 4f to be engaged with the threaded nut 6a is formed on the outer circumference thereof.

In the above-mentioned structure, the threaded nut 4e of the direct-vision attachment 4 is screwed and fixed to the thread 1d of the top end hard portion 1, and then, the anchoring ring 6 is screwed to the thread 4f of the attachment 4, whereby the attachment 4 is fixed and integrated with the top end hard portion 1.

In the above-mentioned structure of the third embodiment, the arrangements of the top end of the top end hard portion 1 and the attachment 4 can be reversed. Also in this case, the above effects are similarly attained.

As is apparent from the foregoing description, according to the present device, the small-diameter and large-diameter thread are inverse to each other or are cut in the same direction at different pitches. If this structure is adopted, when the attachment is attached to the top end hard portion of the endoscope, the attachment is directly screwed to the top end hard portion and is anchored by the anchoring ring. Therefore, even if the anchoring ring comes off, the attachment is kept screwed and falling of the attachment is prevented.

Furthermore, even if the anchoring ring is freely set on the top end hard portion, the effect of preventing falling of the attachment is similarly attained by the double-lock structure.

Moreove, in case of the side-vision attachment, if the anchoring ring comes off, the visual field is disturbed by the anchoring ring, an operator can be informed of coming-off of the anchoring ring and occurrence of an accident can be prevented.

Still further, since the small-diameter and large-diameter threads are inverse to each other or are cut in the same direction at different pitches, even if there are motions in one direction of the endoscope, the anchoring ring and the attachment are prevented to simultaneously come off under the same conditions, and therefore, an assured fixation state can be expected.

## Claims

1. An endoscope comprising a direct-vision attachment (4) and a side-vision attachment (5) which are exchangeably attachable to an object lens (3a) on the top end hard portion of the endoscope, the attachment (4,5) having a threaded nut (4a,5a) formed on the inner side of its rear end and an anchoring ring (6) having a threaded nut (6a) formed on the inner side of its top end,
characterized in that the top end hard portion comprises a thread (1b) having a first diameter, followed, seen from the top end, by a thread (1c) having a second diameter which is larger than the first one; the attachment comprises a flange (4d,5f) at the rear end in the vicinity of the threaded nut (4a,5a); and the anchoring ring (6) is slidably movable towards the flange (4d,5f) of the attachment and comprises an anchoring portion (6b), formed on the inner side of its rear end, for anchoring the flange (4d,5f) of the attachment when the threaded nut (6a) of the anchoring ring (6) is in engagement with the second diameter thread (1c) and the threaded nut (4a,5a) of the attachment is in engagement with the first diameter thread (1b).

2. An endoscope as set forth in claim 1,
characterized in that the first thread (1b) and the second thread (1c) are inverse to each other or cut in the same direction at different pitches.

3. An endoscope comprising a direct-vision attachment (4) and a side-vision attachment (5) which are exchangeably attachable to an object lens (3a) on the top end hard portion of the endoscope, the attachment (4,5) having a threaded nut (4e) formed on the inner side of its rear end, the top end hard portion comprising a thread (1d), followed by a flange (le), and an anchoring ring (6) having a threaded nut (6a), formed on the inner side of its top end, so that the anchoring ring is slidably movable towards the flange (1e),
characterized in that the attachment comprises a thread (4f) on the outer circumference at its rear end to be engaged with the threaded nut (6a) of the anchoring ring (6) when the threaded nut (4e) of the attachment is in engagement with the thread (1d) of the endoscope's top end hard portion.

4. An endoscope as set forth in claim 3,
characterized in that the thread (1d) of the endoscope's top end hard portion and the threaded nut (6a) of the anchoring ring (6) are inverse to each other or cut in the same direction at different pitches.

## Patentansprüche

1. Endoskop mit einem Direktsicht-Beobachtungs-Vorsatz (4) und einem Seitensicht-Beobachtungs-Vorsatz (5), die auswechselbar an einer Objektivlinse (3a) am Kopfende des Endoskops festgelegt sind, wobei die Vorsätze (4, 5) eine Gewindemutter (4a, 5a) auf der Innenseite ihres hinteren Endes und einen Verankerungsring (6) aufweisen, der eine Gewindemutter (6a) an der Innenseite seines Kopfendes aufweist,
dadurch gekennzeichnet, daß das Kopfende des Endoskops ein Gewinde (1b) mit einem ersten Durchmesser aufweist, dem, vom Kopfende her betrachtet, ein Gewinde (lc) mit einem zweiten Durchmesser folgt, der größer ist als der erste Durchmesser; wobei der Vorsatz einen Flansch (4d, 5f) in der Nähe des hinteren Endes benachbart zur Gewindemutter (4a, 5a) aufweist, und daß der Verankerungsring (6) gleitbar nach dem Flansch (4d, 5f) des Vorsatzes hin beweglich ist und einen Verankerungsansatz (6b) auf der Innenseite seines hinteren Endes aufweist, um den Flansch (4d, 5f) des Vorsatzes zu verankern, wenn die Gewindemutter (6a) des Verankerungsrings (6) in Eingriff mit dem Gewinde (1c) mit zweitem Durchmesser steht und die Gewindemutter (4a, 5a) des Vorsatzes in Eingriff mit dem Gewinde (1b) mit erstem Durchmesser steht.

2. Endoskop nach Anspruch 1,
dadurch gekennzeichnet, daß das erste Gewinde (1b) und das zweite Gewinde (1c) invers zueinander mit entgegengesetztem Richtungssinn oder mit gleichem Richtungssinn und unterschiedlicher Steigung ausgebildet sind.

3. Endoskop mit einem Direktsicht-Beobachtungs-Vorsatz (4) und einem Seitensicht-Beobachtungs-Vorsatz (5), die auswechselbar an einer Objektivlinse (3a) am Kopfende des Endoskops angeordnet sind, wobei die Vorsätze (4, 5) eine Gewindemutter (4e) auf der Innenseite ihres hinteren Endes aufweisen und das Kopfende des Endoskops ein Gewinde (1d) besitzt, dem ein Flansch (1e) folgt, wobei ein Verankerungsring (6) mit einer Gewindemutter (6a) an der Innenseite des Kopfendes derart angeordnet ist, daß der Verankerungsring gleitbar auf den Flansch (1b) hin beweglich ist,
dadurch gekennzeichnet, daß jeder Vorsatz ein Gewinde (4f) am äußeren Umfang an seinem hinteren Ende aufweist, mit dem die Gewindemutter (6a) des Verankerungsringes (6) in Eingriff steht, wenn die Gewindemutter (4e) des Vorsatzes in Eingriff mit dem Gewinde (1d) am Kopfende des Endoskops steht.

4. Endoskop nach Anspruch 3,
dadurch gekennzeichnet, daß das Gewinde (1d) am Kopfende des Endoskops und die Gewindemutter (6a) des Verankerungsringes (6) invers zueinander mit entgegengesetztem Richtungssinn oder mit gleichem Richtungssinn und unterschiedlicher Steigung verlaufen.

## Revendications

1. Endoscope comprenant un accessoire (4) pour vision directe et un accessoire (5) pour vision latérale qui peuvent être fixés de façon interchangeable à une lentille (3a) d'objectif sur la partie dure d'extrémité supérieure de l'endoscope, l'accessoire (4, 5) comportant une partie filetée (4a, 5a) formée sur le côté intérieur de son extrémité arrière et une bague de fixation (6) comportant une partie (6a) formée sur le coté intérieur de son extrémité supérieure,
caractérisé en ce que la partie dure d'extrémité supérieure comprend un filetage (1b) ayant un premier diamètre, suivi, vu depuis l'extrémité supérieure, par un filetage (1c) ayant un second diamètre qui est plus grand que le premier diamètre; l'accessoire auxiliaire comprend une collerette (4d, 5f) à l'extrémité arrière au voisinage de la partie filetée (4a, 5a); et la bague de fixation (6) peut coulisser en direction de la collerette (4d, 5f) de l'accessoire et comprend une partie de fixation (6b), formée sur le côté intérieur de son extrémité arrière, pour fixer la collerette (4d, 5f) de l'accessoire quand la partie filetée (6a) de la bague de fixation (6) est vissée sur le filetage (1c) de second diamètre et la partie filetée (4a, 5a) de l'accessoire est vissée sur le filetage (1b) de premier diamètre.

2. Endoscope selon la revendication 1, caractérisé en ce que le premier filetage (1b) et le second filetage (1c) sont l'inverse l'un de l'autre ou sont taillés dans la même direction avec des pas différents.

3. Endoscope comprenant un accessoire (4) pour vision directe et un accessoire (5) pour vision latérale qui peuvent être fixés de façon interchangeable à une lentille (3a) d'objectif sur la partie dure d'extrémité supérieure de l'endoscope, l'accessoire (4, 5) comportant une partie filetée (4e) formée sur le côté intérieur de son extrémité arrière, la partie dure d'extrémité supérieure comprenant un filetage (1d), suivi par un collet (1e), et une bague de fixation (6) comportant une partie filetée (6a), formée sur le côté intérieur de son extrémité supérieure, de sorte que la bague de fixation peut coulisser en direction du collerette (1e),
caractérisé en ce que l'accessoire comprend sur la circonférence extérieure, à son extrémité arrière, un filetage (4f) destiné à être vissé dans la partie filetée (6a) de la bague de fixation (6) quand la partie filetée (4e) de l'accessoire est vissée sur le filetage (1d) de la partie dure d'extrémité supérieure de l'endoscope;

4. Endoscope selon la revendication 3,
caractérisé en ce que le filetage (1d) de la partie dure d'extrémité supérieure de l'endoscope et la partie filetée (6a) de la bague de fixation (6) sont l'inverse l'un de l'autre ou sont taillés dans la même direction avec des pas différents.
